# EUROPEAN PATENT SPECIFICATION

(11) **EP 1 969 947 B1**
(45) Date of publication and mention of the grant of the patent: **22.12.2010**
(21) Application number: 08075085.4
(22) Date of filing: 06.02.2008
(51) Int. Cl.: A23C 3/037, A23L 3/22, A61L 2/07

(54) **A steam injector for direct heating of flowing materials**
Dampfeinspritzer zur Direkterhitzung fließender Materialien
Injecteur de vapeur pour le chauffage direct de matériaux de flux

(30) Priority: 09.03.2007 IT MO20070078
(43) Date of publication of application: 17.09.2008
(73) Proprietor: CFT S.p.A., 43100 Parma (IT)
(72) Inventor: Catelli Roberto, 43100 Parma (IT); Lazzari, Alessio, 46030 Viadana, Mantova (IT)
(74) Representative: Gotra, Stefano

(56) References cited:
- EP-A- 0 210 887
- FR-A- 1 114 013
- GB-A- 413 460
- GB-A- 2 036 534
- US-A- 4 851 250

## Description

The invention relates to a steam injector, especially an injector used in the food or chemical industry for directly heating flowing materials.

In the example, the injector of the present invention is advantageously used in process plants for milk sterilisation according to the UHT system.

As is known, in order to treat milk such as to obtain long-life milk a normal pasteurisation process is not sufficient, as it does not deactivate the spores in the product. The production of long-life milk occurs via sterilisation using the U.H.T. (Ultra High Temperature) process, which, very briefly, includes heating the product to a temperature of around 150°C for a very short time before packing the product inside previously-sterilised containers.

In processes of this type, the product must be maintained at the highest process temperature for as short a time as possible, given that the quality of the product can deteriorate if subjected to excessive times at the top temperature.

To enable the process temperature to be maintained for short periods, direct heating methods of the product to be processed are used, i.e. the product is placed directly in contact with steam of a quality suitable for food use.

High-pressure steam is injected into a conduit through which the milk flows at a temperature of about 80°C. The operation causes a sharp raising of the product temperature (up to about 150°C), which is then taken to an instant refrigeration station where it is separated from the injected steam.

A steam injector which is suitable for use in a process of the present type is described in UK patent GB 2389576. The steam injector comprises a steam injection chamber containing a series of holed discs piled one on another to define a central diffusion conduit in which the product to be subjected to direct heating flows. The discs are provided with channels which place the central hole in communication with the external periphery, such that the assembled diffusion conduit is in communication with the rest of the injection chamber, which contains pressurised steam during the normal functioning of the device. The discs are held together by two con rods screwed to plates which inferiorly and superiorly close the pile.

The device described, however, exhibits some drawbacks connected to the presence of the superposed discs.

The device is sterilised using flowing steam at a temperature of 170°C. The volume expansion induced in the discs on the increase of temperature is not accompanied by the dilation of the (metal) con rods, as the metal has a heat dilation coefficient which is much lower than that of the plastic material used for making the discs. Consequently, with the pile of the discs being kept compressed, the heat expansion of the single disc is translated into a variation of the diameters with constant thickness. When the disc is brought back to the process temperature of 150°C, the element shrinks. As the plastics used in the manufacturing of the discs are not of a shape-memory type, the shrinkage influences all the dimensions of the disc, including a reduction in the thickness. An obvious negative consequence of this change in thickness is the loss of adherence between the discs, with a consequent loss of seal in the diffusion conduit.

What is more, the high number of elements to be assembled leads to high mounting/dismounting times, which translates into lengthy maintenance halts with a consequent reduction of mean process plant throughput where the plant comprises the device.

Document GB 413460 discloses a pasteuriser for lacteal liquids comprising a container having a means enabling the liquid to be passed therethrough in a thin film or in a finely divided state and means for gently intermingling or diffusing steam with the liquid to raise said liquid to pasteurising temperature, wherein the means for gently diffusing steam into said film of liquid comprises providing the container with a plurality of small holes therein.

The aim of the present invention is to obviate the drawbacks of the prior art by realising a steam adaptor suitable for use in sterilisation processes for flowing food products which exhibits excellent characteristics of resistance to heat fatigue in the diffusion conduit.

An advantage of the invention is the simplicity and rapidity of the mounting and dismounting stages for maintaining the device.

A further advantage of the invention is constituted by the simplicity and economic aspects of the device.

These aims and advantages and more besides are all attained by the present invention, as it is characterised in the appended claims.

Further characteristics and advantages will better emerge from the detailed description that follows of a preferred but not exclusive embodiment of the device, illustrated purely by way of non-limiting example in the accompanying figures of the drawings, in which:

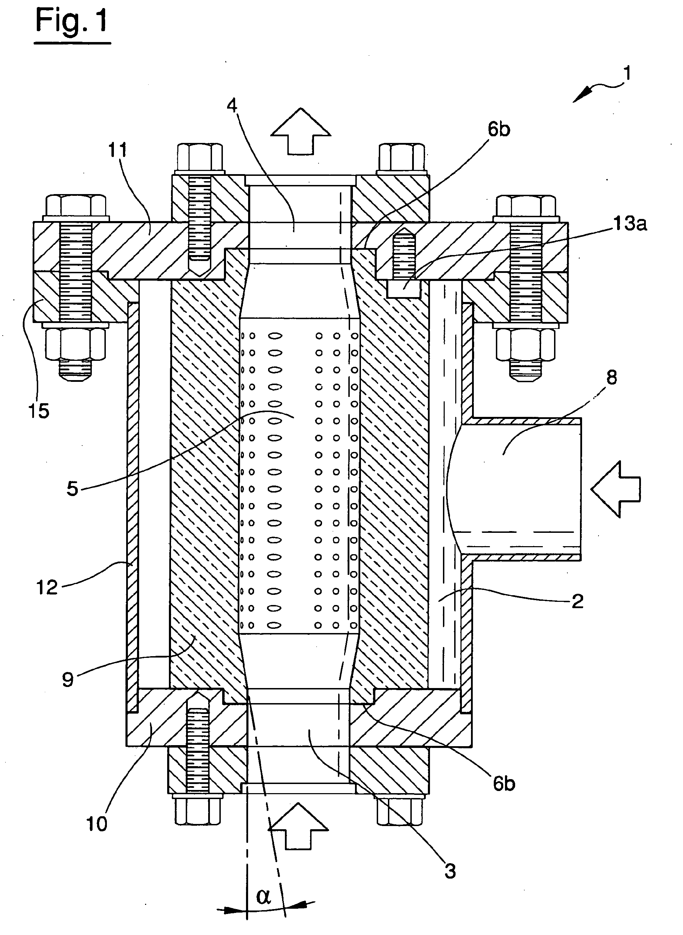

figure 1 is a section view of the steam injector of the invention;

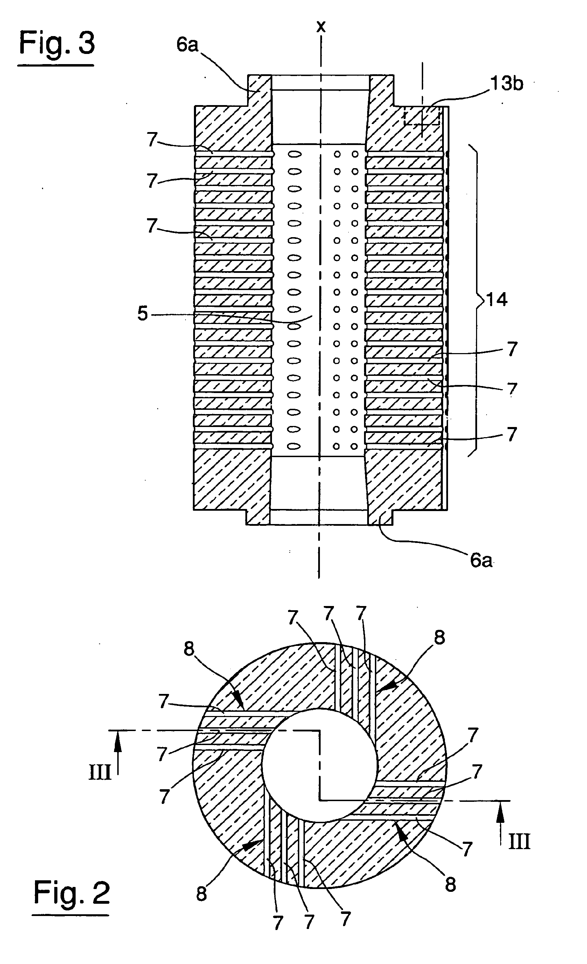

figure 2 is a plan view of the single-piece central tubular element of the steam injector of the present invention;
figure 3 is the section view along line II-II in figure 2 of the single-piece central tubular element of the steam injector of the present invention.

With reference to the figures of the drawings, 1 denotes a steam injector for direct heating of flowing material, comprising an injection chamber 2 provided with an inflow opening 3 and an outflow opening 4 for the flowing material to be heated and a diffusion conduit 5, internally of the injection chamber 2, which directly connects the two openings.

The injection chamber 2 is further provided with a third opening 8, associable to an external source of steam. During normal functioning the source of steam, of known type, enables the device to maintain pressurised steam internally of the chamber 2.

The walls of the diffusion conduit are provided with a plurality of channels 7 which place the diffusion conduit 5 in communication with the rest of the injection chamber 2. The channels enable transit of the pressurised steam from the chamber 2 to the conduit 5, which steam is condensed into the milk (or another product to be sterilised), heating it up to a temperature which causes sterilisation thereof.

To obviate the drawbacks of the above-described known process, the walls of the diffusion conduit 5 are not made from a pile of superposed rings, but a single-piece tubular element 9.

In the preferred embodiment, the injection chamber is delimited by a bottom plate 10 and a top plate 11, opposite one another and constrained by a cylindrical casing 12. The two plates 10, 11 are constituted by circular plates. The bottom plate 10 centrally exhibits a through-hole which realises the inflow opening 3; correspondingly, the top plate 11 exhibits the outflow opening 4 at a centre thereof. The third opening 8 is advantageously laterally-located, on the cylindrical casing 12. The two plates 10, 11 and the cylindrical casing 12 are preferably made of steel.

The single-piece tubular element 9, which delimits the diffusion conduit 5, substantially exhibits a hollow cylindrical shape, with the channels 7 placing in communication the external and internal surfaces thereof.

The channels 7 develop linearly along at least a transversal section of the single-piece tubular element 9.

Considering a transversal section, the channels which develop on the section are organised in various groups 8 having central symmetry with respect to the longitudinal axis x of the diffusion conduit 5. Each group comprises at least a channel. The channels of a same group 8 are parallel to one another and at least a channel of each group 8 develops along a substantially skewed axis with respect to the longitudinal axis x of the diffusion conduit 5.

A first channel 7 of each group 8 is preferably substantially tangential on the internal surface of the single-piece tubular element 9. The other channels 7 of the group 8 are parallel and contiguous to the first channel, and their position with respect to the internal surface of the single-piece tubular element 9 is in any case more tangential than normal. This characteristic, together with the central symmetry of the groups 8 of conduits 7 which symmetry means that to each group a second group is associated, facing in a diametrically opposite position opposite the first group on the internal surface of the single-piece tubular element 9, is such that the injected steam determines a considerable turbulent action on the product flowing in the diffusion conduit 5. The turbulent motion enables an effective sterilisation action.

In the embodiment illustrated in the above-mentioned figures of the drawings, the channels 7 develop along a plurality of equi-spaced sections, and twelve channels develop on each section, the channels being subdivided into four groups 8 (each formed by three channels). Other embodiments preferably comprise a number of channels from between four and sixteen, but always divided into four groups 8.

The single-piece tubular element 9 described is arranged internally of the injection chamber 2 and connects the inflow opening 3 and the outflow opening 4.

The ends of the single-piece tubular element 9 are counter-shaped with respect to the internal surfaces of the two plates 10, 11 in order to be able to house the single-piece tubular element internally of the injection chamber 2 and prevent it from rotating.

In the example, the single-piece tubular element 9 comprises two cylindrical projections 6a at the two ends thereof, which projections 6a are coaxial to the longitudinal axis x of the diffusion conduit 5, and which projections 6a are predisposed to be housed internally of two cylindrical recesses 6b afforded on the internal surfaces of the two plates 10, 11.

The bottom plate 10 is preferably peripherally welded to the bottom end of the cylindrical casing 12. The upper end of the cylindrical casing 12 is welded to an annular flange 15, predisposed to be bolted to the upper plate 11, peripherally flanged.

The mounting of the device is performed simply and rapidly, housing a cylindrical projection 6a of the single-piece tubular element 9 in the cylindrical recess of the bottom plate 10, then closing off the injection chamber 2 by applying the upper plate 11 and bolting the flanged ring 15.

Given the central symmetry of the cylindrical projections 6a and the corresponding recesses 6b, even when fastened between the two plates, the single-piece tubular element 9 is freely rotatable about the axis thereof with respect to the injection chamber 2.

To obviate this drawback, the device comprises at least a pivot 13a, solidly constrained to one of the plates 11, the pivot being predisposed to engage internally of a cavity 13b afforded on the surface of the single-piece tubular element in contact with the plate.

Note how in the described embodiment, in order to enable an effective steam injection action, the diffusion conduit 5 preferably exhibits a variable-diameter circular section. The diameter is greater in a central tract 14 of the conduit, on which the majority of the channels 7 open, and smaller in the tracts at the inflow 3 and outflow 4 openings. The tracts of larger and smaller section are connected to one another by truncoconical chokes, which thus prevent any sharp variations in the conduit's section.

The internal walls of the single-piece tubular element 9 at the truncoconical chokes of the diffusion conduit 5 are inclined with respect to the walls of the central tract 14 by an angle α which is less than 10 degrees and which in the preferred embodiment is 7.5 degrees.

The single-piece tubular element 9 is made of a plastic material which is resistant to heat, preferably polytetrafluoroethylene. It could also be made of polychlorotrifluoroethylene.

The described device, as previously mentioned, can be applied to a plant for sterilising flowing food products, and can in particular be applied in sterilisation of the milk in a UHT process.

## Claims

1. A steam injector (1) for direct heating of flowing material, comprising:
an injection chamber (2) provided with an inflow opening (3) and an outflow opening (4) for flowing material to be heated, as well as a third opening (8) associable to a source of steam external of the chamber, a diffusion conduit (5), internal of the injection chamber (2), which directly connects the inflow opening (3) with the outflow opening (4), the walls of the diffusion conduit being provided with a plurality of channels (7) which place the diffusion conduit (5) in communication with the rest of the injection chamber (2), the walls of the diffusion conduit being constituted by a single-piece tubular element (9);
**characterised in that** the channels (7) develop linearly along at least a transversal section of the single-piece tubular element (9), the channels developing along each transversal section being organised in groups (8) comprising at least a channel, channels of a same group (8) being parallel to one another, at least a channel of each group (8) having a development axis which is substantially skewed with respect to the longitudinal axis (x) of the diffusion conduit (5), the groups (8) being provided with a central symmetry with respect to the longitudinal axis (x) of the diffusion conduit (5).

2. The steam injector of claim 1, **characterised in that** the channels (7) develop along a plurality of equi-spaced sections, twelve channels, subdivided into four groups (8), developing on each section.

3. The steam injector of one of the preceding claims, **characterised in that** the injection channel (2) is delimited by two plates (10, 11) which are opposite and constrained by a cylindrical casing (12), ends of the single-piece tubular element (9) being counter-shaped with respect to the internal surfaces of the two plates (10, 11) in order to enable housing the single-piece tubular element (9) internally of the injection chamber (2).

4. The steam injector of claim 3, **characterised in that** the single-piece tubular element (9) comprises, at the ends thereof, two cylindrical projections (6a), coaxial to the longitudinal axis (x) of the diffusion conduit (5), which cylindrical projections (6a) are predisposed to be housed in two cylindrical recesses (6b) exhibited in internal surfaces of the two plates (10, 11).

5. The steam injector of claim 4, **characterised in that** it comprises at least a pivot (13a), solidly constrained to a plate (11) of the two plates (10, 11), the pivot (13a) being predisposed to engage internally of a cavity (13b) exhibited on the surface of the single-piece tubular element (9) in contact with the plate (10) in order solidly to constrain the tubular element (9) in rotation with the plate (11).

6. The steam injector of one of the preceding claims, **characterised in that** the diffusion conduit (5) exhibits a variable-diameter circular section, the diameter being greater in a central tract (14) of the conduit, on which central tract (14) a majority of the channels (7) open, and the diameter being smaller in tracts near the inflow opening (3) and the outflow opening (4), the greater- and smaller-diameter tracts being connected to one another by means of truncoconical chokes.

7. The steam injector of claim 6, **characterised in that** the internal walls of the single-piece tubular element (9), at the truncoconical chokes of the diffusion conduit (5), are inclined with respect to the walls of the central tract (14) by an angle α which is smaller than 10 degrees.

8. The steam injector of one of the preceding claims, **characterised in that** the single-piece tubular element (9) is made of a plastic material which is resistant to heat.

9. The steam injector of claim 8, **characterised in that** the single-piece tubular element (9) is made of polytetrafluoroethylene.

## Patentansprüche

1. Dampfeinspritzer (1) zur Direkterhitzung von fliessenden Materialien, enthaltend: eine Einspritzkammer (2), versehen mit einer Zuflussöffnung (3) und einer Abflussöffnung (4) für das zu erhitzende fliessende Material, sowie eine dritte Öffnung (8), zuzuordnen einer Dampfquelle ausserhalb der Kammer, eine Verteilerrohrleitung (5) im Inneren der Einspritzkammer (2), welche direkt die Zuflussöffnung (3) mit der Abflussöffnung (4) verbindet, wobei die Wände der Verteilerrohrleitung mit einer Anzahl von Kanälen (7) versehen sind, welche die Verteilerrohrleitung (5) mit dem Rest der Einspritzkammer (2) in Verbindung bringen, wobei die Wände der Verteilerrohrleitung in einem Stück aus einem rohrförmigen Element (9) gebildet sind; **dadurch gekennzeichnet, dass** die Kanäle (7) sich linear entlang von wenigstens einem querverlaufenden Abschnitt des aus einem Stück gebildeten rohrförmigen Elementes (9) erstrecken, wobei die sich entlang einem jeden querverlaufenden Abschnitt erstreckenden Kanäle in jeweils wenigstens einen Kanal enthaltenden Gruppen (8) zusammengefasst sind, wobei die Kanäle einer selben Gruppe (8) parallel zueinander verlaufen, wobei wenigstens ein Kanal einer jeden Gruppe (8) eine Ausdehnungsachse hat, die im wesentlichen schräg im Verhältnis zu der Längsachse (x) der Verteilerrohrleitung (5) ist, und wobei die Gruppen (8) mit einer mittleren Symmetrie im Verhältnis zu der Längsachse (x) der Verteilerrohrleitung (5) versehen sind.

2. Dampfeinspritzer nach Patentanspruch 1, **dadurch gekennzeichnet, dass** sich die Kanäle (7) entlang einer Anzahl von gleichmässig voneinander abstehenden Abschnitten erstrecken, und zwar zwölf Kanäle in jedem Abschnitt, unterteilt in vier Gruppen (8).

3. Dampfeinspritzer nach einem der vorstehenden Patentansprüche, **dadurch gekennzeichnet, dass** die Einspritzkammer (2) durch zwei Platten (10, 11) abgegrenzt ist, die sich gegenüberliegen und durch ein zylindrisches Gehäuse (12) gehalten sind, wobei die Enden des aus einem Stück gebildeten rohrförmigen Elementes (9) im Verhältnis zu den Innenflächen der beiden Platten (10, 11) gegengeformt sind, um die Aufnahme des aus einem Stück gebildeten rohrförmigen Elementes (9) im Inneren der Einspritzkammer (2) zu ermöglichen.

4. Dampfeinspritzer nach Patentanspruch 3, **dadurch gekennzeichnet, dass** das aus einem Stück gebildete rohrförmige Element (9) an seinem Enden zwei zylindrische Vorsprünge (6a) aufweist, koaxial zu der Längsachse (x) der Verteilerrohrleitung (5), welche zylindrischen Vorsprünge (6a) dazu bestimmt sind, in zwei zylindrischen Vertiefungen (6b) aufgenommen zu werden, aufgewiesen in den Innenflächen der beiden Platten (10, 11).

5. Dampfeinspritzer nach Patentanspruch 4, **dadurch gekennzeichnet, dass** er wenigstens einen Zapfen (13a) enthält, fest gehalten an einer Platte (11) der beiden Platten (10, 11), wobei der Zapfen (13a) dazu bestimmt ist, in das Innere einer Vertiefung (13b) zu greifen, die an der sich im Kontakt mit der Platte (10) befindlichen Oberfläche des aus einem Stück gebildeten rohrförmigen Elementes (9) vorhanden ist, um das rohrförmige Element (9) in der Umdrehung fest an der Platte (10) zu halten.

6. Dampfeinspritzer nach einem der vorstehenden Patentansprüche, **dadurch gekennzeichnet, dass** die Verteilerrohrleitung (5) einen Kreisquerschnitt mit veränderbarem Durchmesser aufweist, wobei der Durchmesser in einem mittleren Abschnitt (14) der Leitung grösser ist, an welchem mittleren Abschnitt (14) die Mehrzahl der Kanäle (7) mündet, und der Durchmesser in den Abschnitten dicht an der Zuflussöffnung (3) und der Abflussöffnung (4) kleiner ist, und wobei die Abschnitte mit den grösseren und kleineren Durchmessern mit Hilfe durch stumpfkegelförmige Drosselstellen miteinander verbunden sind.

7. Dampfeinspritzer nach Patentanspruch 6, **dadurch gekennzeichnet, dass** die Innenwände des aus einem Stück gebildeten rohrförmigen Elementes (9) an den stumpfkegelförmigen Drosselstellen der Verteilerrohrleitung (5) im Verhältnis zu den Wänden des mittleren Abschnittes (14) um einen Winkel α geneigt sind, welcher kleiner ist als 10 Grad.

8. Dampfeinspritzer nach einem der vorstehenden Patentansprüche, **dadurch gekennzeichnet, dass** das aus einem Stück gebildete rohrförmige Element (9) aus hitzbeständigem Kunststoffmaterial hergestellt ist.

9. Dampfeinspritzer nach Patentanspruch 8, **dadurch gekennzeichnet, dass** das aus einem Stück gebildete rohrförmige Element (9) aus Polytetrafluoräthylen hergestellt ist.

## Revendications

1. Injecteur de vapeur (1) pour le chauffage direct de matériaux de flux, comprenant: une chambre d'injection (2) pourvue d'une ouverture d'entrée (3) et d'une ouverture de sortie (4) pour le matériau de flux à chauffer, ainsi qu'une troisième sortie (8) associable à une source de vapeur externe à la chambre, un conduit de diffusion (5), interne à la chambre d'injection (2), qui relie directement l'ouverture d'entrée (3) à l'ouverture de sortie (4), les parois du conduit de diffusion (5) étant pourvues d'une pluralité de canaux (7) qui mettent le conduit de diffusion (5) en communication avec le reste de la chambre d'injection (2), les parois du conduit de diffusion étant constituées d'un élément tubulaire monobloc (9);
**caractérisé en ce que** les canaux (7) se développent linéairement le long d'au moins une section transversale de l'élément tubulaire monobloc (9), les canaux se développant le long de chaque section transversale étant organisés en groupes (8) comprenant au moins un canal, les canaux d'un même groupe (8) étant parallèles entre eux, au moins un canal de chaque groupe (8) ayant un axe de développement substantiellement de travers par rapport à l'axe longitudinal (x) du conduit de diffusion (5), les groupes (8) étant dotés d'une symétrie centrale par rapport à l'axe longitudinal (x) du conduit de diffusion (5).

2. Injecteur de vapeur selon la revendication 1, **caractérisé en ce que** les canaux (7) se développent le long d'une pluralité de sections équidistantes, douze canaux, subdivisés en quatre groupes (8), se développant sur chaque section.

3. Injecteur de vapeur selon l'une quelconque des revendications précédentes, **caractérisé en ce que** la canal d'injection (2) est délimité par deux plaques (10, 11) opposées et engagées par un carter cylindrique (12), les extrémités de l'élément tubulaire monobloc (9) étant contre-profilées par rapport aux surfaces internes des deux plaques (10, 11) de manière à permettre le logement de l'élément tubulaire monobloc (9) à l'intérieur de la chambre d'injection (2).

4. Injecteur de vapeur selon la revendication 3, **caractérisé en ce que** l'élément tubulaire monobloc (9) comprend, en correspondance de ses extrémités, deux saillies tubulaires (6a), coaxiales à l'axe longitudinal (x) du conduit de diffusion (5), lesquelles saillies cylindriques (6a) étant prédisposées pour être logées dans deux renfoncements cylindriques (6b) formés dans les surfaces internes des deux plaques (10, 11).

5. Injecteur de vapeur selon la revendication 4, **caractérisé en ce qu'**il comprend au moins un pivot (13a), solidaire d'une plaque (11) des deux plaques (10, 11), le pivot (13a) étant prédisposé pour engager intérieurement une cavité (13b) présentée sur la surface de l'élément tubulaire monobloc (9) en contact avec la plaque (10) de manière à rendre l'élément tubulaire (9) solidaire en rotation de la plaque (11).

6. Injecteur de vapeur selon l'une des précédentes revendications, **caractérisé en ce que** le conduit de diffusion (5) présente une section circulaire de diamètre variable, le diamètre étant supérieur dans une zone centrale (14) du conduit, sur laquelle zone centrale (14) débouchent une majorité des canaux (7), et le diamètre étant inférieur dans des zones à proximité de l'ouverture d'entrée (3) et de l'ouverture de sortie (4), les zones de diamètre supérieur et de diamètre inférieur étant reliées entre elles au moyen d'étranglements en tronc de cône.

7. Injecteur de vapeur selon la revendication 6, **caractérisé en ce que** les parois latérales de l'élément tubulaire monobloc (9), en correspondance des étranglements en tronc de cône du conduit de diffusion (5), sont inclinées par rapport aux parois de la zone centrale (14) selon un angle α inférieur à 10 degrés.

8. Injecteur de vapeur selon l'une des revendications précédentes, **caractérisé en ce que** l'élément tubulaire monobloc (9) est réalisé en un matériau plastique résistant à la chaleur.

9. Injecteur de vapeur selon la revendication 8, **caractérisé en ce que** l'élément tubulaire monobloc (9) est réalisé en polytétrafluoroéthylène.
